# EUROPEAN PATENT APPLICATION

(11) **EP 4 696 275 A1**
(43) Date of publication of application: **18.02.2026**
(21) Application number: 24806245.7
(22) Date of filing: 11.04.2024
(51) Int. Cl.: A61B 34/30

(54) **DRIVING DEVICE, STERILIZATION BARRIER, AND SURGICAL ROBOT**

(30) Priority: 15.05.2023 CN 202310545803
(71) Applicant: Ronovo (Shanghai) Medical Science and Technology Ltd., Shanghai 201102 (CN)
(72) Inventor: SU, Zhiqiang, Shanghai 201102 (CN)
(74) Representative: Cabinet Beaumont
(86) International application number: PCT/CN2024/087134
(87) International publication number: WO 2024/234886

(57) **Abstract**

A drive device is configured to drive a laparoscope (7) to rotate and includes an outer housing (1), an inner housing (2), and a drive assembly (3). The inner housing (2) is rotatably connected to the outer housing (1) via a bearing (5), the inner housing (2) forms a first mounting cavity configured for mounting a sterile barrier, the laparoscope (7) penetrates through the sterile barrier, and a second mounting cavity is formed between an outer wall of the inner housing (2) and an inner wall of the outer housing (1). The drive assembly (3) is disposed in the second mounting cavity and connected to the inner housing (2) to drive the inner housing (2) to rotate.

## Description

This application claims priority to Chinese Patent Application No. 202310545803.4 filed with the China National Intellectual Property Administration (CNIPA) on May 15, 2023, the disclosure of which is incorporated herein by reference in its entirety.

### TECHNICAL FIELD

The present application relates to the technical field of medical devices, for example, a drive device, a sterile barrier, and a surgical robot.

### BACKGROUND

Due to the applications of surgical robots in the medical field, many problems that are difficult to solve through manual operations have been solved. For example, a laparoscope on a surgical robot can extend into a patient's body to provide a surgeon with a visual field, making it convenient for the surgeon to perform the corresponding operations.

In the related art, the laparoscope on the surgical robot is disposed at an end of a robotic arm and connected to a drive device in the robotic arm so that the laparoscope is powered by the drive device to perform the corresponding actions, such as rotation or translation, to smoothly provide a field for the surgeon. The installation of the laparoscope requires not only a sterile environment but also the adaptability with the robotic arm. Therefore, two structures, a sterile adapter and a coupling adapter, are generally provided between the laparoscope and the robotic arm.

However, due to the addition of the sterile adapter and the coupling adapter, an electric motor in a drive box is required to be connected to the sterile adapter and the coupling adapter before being connected to the laparoscope, resulting in an increase in the length of the whole drive box including the electric motor and affecting the route of the corresponding laparoscope. Thus, a movable range of the laparoscope is reduced, affecting the normal use of the surgical robot.

Additionally, technical solutions in the related art include a drive box that has a small effect on the route of the laparoscope, while the laparoscope cannot achieve continuous rotation beyond 360°.

### SUMMARY

The present application provides a drive device, a sterile barrier, and a surgical robot, thereby solving the problem of a limited movement route of a laparoscope, which affects the normal use of the surgical robot, due to the addition of an adapter and a coupling adapter to the surgical robot during mounting of the laparoscope in the related art.

In a first aspect, the present application provides a drive device configured to drive a laparoscope to rotate. The drive device includes an outer housing, an inner housing, and a drive assembly. The inner housing is rotatably connected to the outer housing via a bearing, the inner housing forms a first mounting cavity configured for mounting a sterile barrier, the laparoscope penetrates through the sterile barrier, and a second mounting cavity is formed between an outer wall of the inner housing and an inner wall of the outer housing. The drive assembly is disposed in the second mounting cavity and connected to the inner housing to drive the inner housing to rotate.

In a second aspect, the present application provides a sterile barrier mounted in the drive device according to the first aspect to fixedly connect the laparoscope to the inner housing and isolate the laparoscope from the inner housing. The sterile barrier includes an isolation assembly penetrating through the first mounting cavity and fixed relative to the inner housing; and a sterile drape disposed on the isolation assembly.

In a third aspect, the present application provides a surgical robot. The surgical robot includes a robotic arm; the drive device according to the first aspect, which is disposed on the robotic arm; and the sterile barrier according to the second aspect, which is disposed in the drive device to enable the laparoscope to be detachably connected to and remain isolated from the drive device.

### BRIEF DESCRIPTION OF DRAWINGS

FIG. 1 is a sectional view of a drive device according to some embodiments of the present application.
FIG. 2 is an exploded view of a drive device according to some embodiments of the present application.
FIG. 3 is a structural view of an inner housing and an outer housing of a drive device according to some embodiments of the present application.
FIG. 4 is a structural view of a bottom wall of a base of a drive device according to some embodiments of the present application.
FIG. 5 is a structural view of a surgical robot according to some embodiments of the present application.

### Reference list:

- 1: outer housing
- 10: conduction opening
- 2: inner housing
- 20: first clamping slot
- 21: second clamping slot
- 22: interface
- 3: drive assembly
- 30: stator
- 31: rotor
- 32: encoder magnetic ring
- 33: encoder read head
- 34: shield ring
- 4: isolation assembly
- 40: first sterile shell
- 400: first sleeve
- 401: clamping block
- 402: snap-fit
- 41: second sterile shell
- 410: sterile plate
- 411: second sleeve
- 412: top ring
- 413: insertion block
- 42: mounting ring-shaped plate
- 420: sterile drape
- 5: bearing
- 50: outer pressure plate
- 51: inner pressure plate
- 6: base
- 60: clamping gap
- 62: plunger
- 63: detector
- 7: laparoscope
- 8: box
- 9: robotic arm

### DETAILED DESCRIPTION

In the description of the present application, terms "joined", "connected", and "fixed" are to be understood in a broad sense unless otherwise expressly specified and limited. For example, the term "connected" may refer to "fixedly connected", "detachably connected", or "integrated", may refer to "mechanically connected" or "electrically connected", may refer to "connected directly" or "connected indirectly through an intermediary", or may refer to "connected inside two components" or "an interaction relation between two components". For those of ordinary skill in the art, meanings of the preceding terms in the present application may be understood according to situations.

In the present application, unless otherwise expressly specified and limited, when a first feature is described as "on" or "below" a second feature, the first feature and the second feature may be in direct contact or be in contact via another feature between the two features instead of being in direct contact. Moreover, when the first feature is described as "on", "above", or "over" the second feature, the first feature is right on, above, or over the second feature, the first feature is obliquely on, above, or over the second feature, or the first feature is simply at a higher level than the second feature. When the first feature is described as "under", "below", or "underneath" the second feature, the first feature is right under, below, or underneath the second feature, the first feature is obliquely under, below, or underneath the second feature, or the first feature is simply at a lower level than the second feature.

In the description of this embodiment, orientations or position relations indicated by terms such as "above", "below", and "right" are based on the drawings. These orientations or position relations are intended only to facilitate description and simplify operations and not to indicate or imply that a device or element referred to must have such particular orientations or must be configured or operated in such particular orientations. Thus, these orientations or position relations are not to be construed as limiting the present application. Additionally, terms "first" and "second" are used only for the distinguishing purpose and have no special meanings.

The present application provides a drive device, a sterile barrier, and a surgical robot. The surgical robot can control the operation of the drive device and drive a laparoscope to rotate through the sterile barrier, and the sterile barrier can keep the laparoscope isolated from the drive device and ensure a sterile environment of the laparoscope. The drive device has the characteristic of a relatively small size, especially a relatively small length dimension in an axial direction of the laparoscope. Thus, an overlap with the laparoscope has a relatively small length dimension so that when the laparoscope is connected to the drive device, the length of the laparoscope is not reduced, ensuring that the laparoscope can move a relatively large route. Meanwhile, the drive device can drive the laparoscope to rotate by an angle greater than 360°. Therefore, during use, the surgical robot can ensure that the laparoscope can move a relatively large route and rotate by a relatively large angle, ensuring that the surgical robot can be normally applied to surgery and improving the success rate of the surgery.

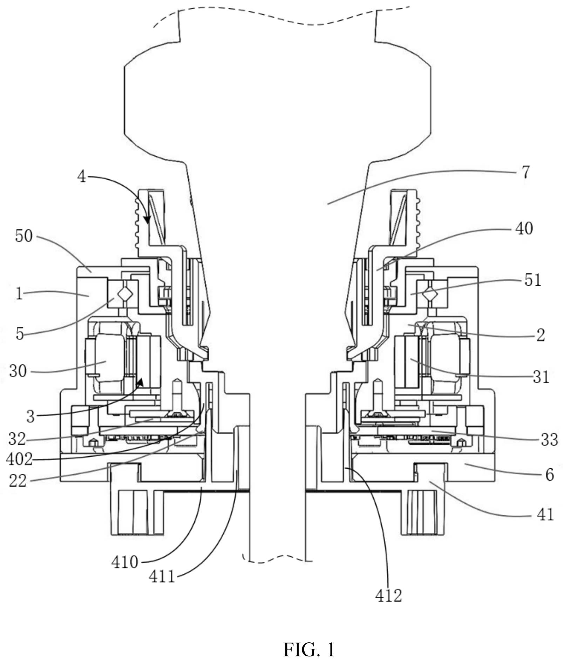

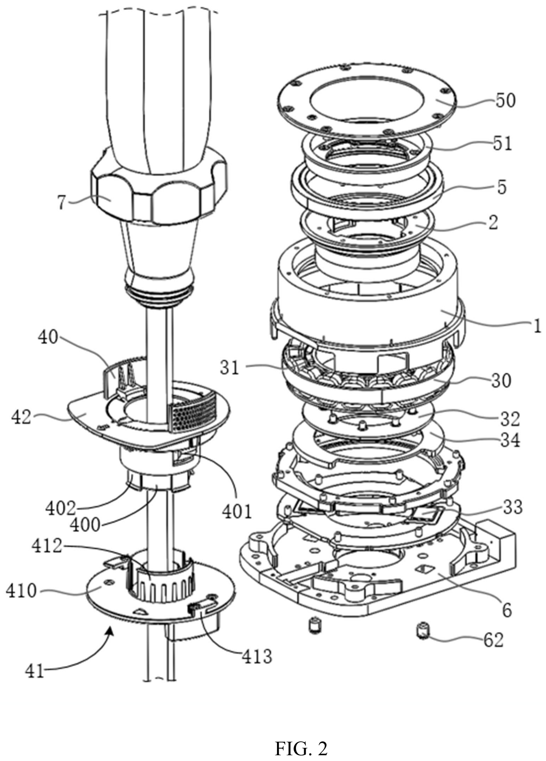

Referring to FIGS. 1 and 2, a drive device according to an embodiment of the present application is configured to drive a laparoscope 7 to rotate and includes an outer housing 1, an inner housing 2, and a drive assembly 3. The inner housing 2 is disposed in the outer housing 1 and rotatably connected to the outer housing 1 via a bearing 5, the inner housing 2 forms a first mounting cavity configured for mounting a sterile barrier, and the laparoscope 7 penetrates through the sterile barrier. A second mounting cavity is formed between an outer wall of the inner housing 2 and an inner wall of the outer housing 1, and the drive assembly 3 is disposed in the second mounting cavity and connected to the inner housing 2 to drive the inner housing 2 to rotate.

For example, the outer housing 1 and the inner housing 2 are both annular, for example, cylindrical, and the inner housing 2 is located inside the outer housing 1. The height of the inner housing 2 is lower than the height of the outer housing 1, a step is formed at the upper end of the inner wall of the outer housing 1, an outer ring of the bearing 5 is supported on an end face of the step, an upper end face of the outer ring of the bearing 5 is slightly lower than an upper end face of the outer housing 1, and the two upper end faces are fixedly connected by an outer pressure plate 50, where the outer pressure plate 50 is fixedly connected to the outer ring of the bearing 5 and the outer housing 1 separately, and the fixing manner may be snap-fit or bolt connection.

A lower end face of an inner ring of the bearing 5 is pressed against an upper end face of the inner housing 2, a step may also be provided on an inner wall of the inner housing 2, and an inner pressure plate 51 with a Z-shaped section may be provided on the step, where the inner pressure plate 51 is fixedly connected to the inner ring of the bearing 5 and the inner housing 2 separately, and the inner pressure plate 51 is fixedly connected to both an inner sidewall and an upper end face of the inner ring of the bearing 5 to fix the inner housing 2 to the inner ring of the bearing 5. The bearing 5 may be a single-row crossed roller bearing and can not only make the inner housing 2 rotatably connected to the outer housing 1 but also implement a certain rigid support function to ensure the connection rigidity between the inner housing 2 and the outer housing 1. Moreover, the bearing 5 has a relatively small axial dimension so that the outer housing 1 and the inner housing 2 are not required to have too large axial dimensions, which is conducive to reducing the overall axial length of the drive device.

The inside of the inner housing 2 is hollow to form the first mounting cavity, the sterile barrier may be divided into two parts which are inserted into the first mounting cavity from the upper end and the lower end of the inner housing 2, respectively, and the sterile barrier may be clamped with the inner housing 2 to remain relatively fixed so that the sterile barrier is detachably connected to the inner housing 2 and forms for the whole robotic arm a sterile environment for mounting and use of the drive device and the laparoscope 7. A sterile channel is formed in the sterile barrier, and the laparoscope 7 penetrates through the sterile channel and is fixed to the sterile barrier through clamping to rotate synchronously with the sterile barrier.

An outer sidewall of the inner housing 2 is stepped, and the outer diameter of a lower part of the inner housing 2 is smaller than the inner diameter of the outer housing 1 so that the second mounting cavity is formed. The drive assembly 3 is disposed in the second mounting cavity and may be fixed to the inner wall of the outer housing 1 and connected to the outer wall of the inner housing 2 to drive the inner housing 2 to rotate. The drive assembly 3 may also be annular and sleeved on the outer sidewall of the inner housing 2, which is entirely annular so that the inner housing 2 can rotate by an angle greater than 360°. Thus, the sterile barrier can rotate synchronously with the inner housing 2, and thus the laparoscope 7 can rotate by an angle greater than 360°.

During use of the drive device, the laparoscope 7 is directly mounted into the first mounting cavity via the sterile barrier, and the drive assembly 3 located in the second mounting cavity may drive the inner housing 2 to rotate to drive the laparoscope 7 to rotate so that when the laparoscope 7 is mounted, an adapter is no longer required, thereby effectively reducing the overall volume and size of the drive device, avoiding interference with a movement route of the laparoscope 7, and enabling the laparoscope 7 to move within a relatively large axial movement range. Meanwhile, the inner housing 2 is connected to the outer housing 1 via the bearing 5 so that when driven by the drive assembly 3, the inner housing 2 can rotate relative to the outer housing 1 by an angle greater than 360° so that the laparoscope 7 can also rotate by such an angle. Therefore, the laparoscope 7 can rotate by a relatively large angle while satisfying a relatively large movement route.

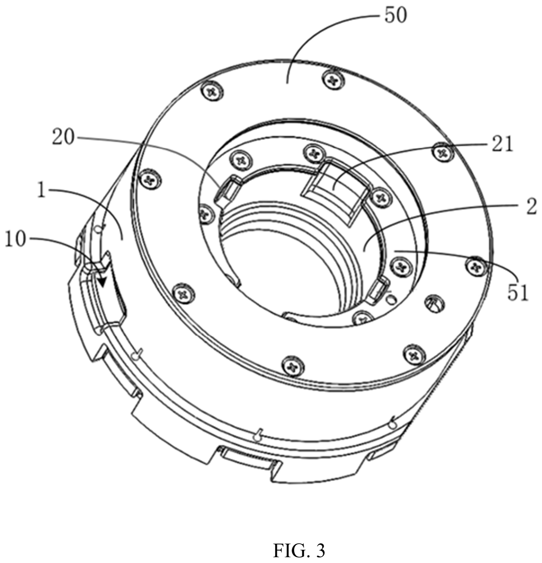

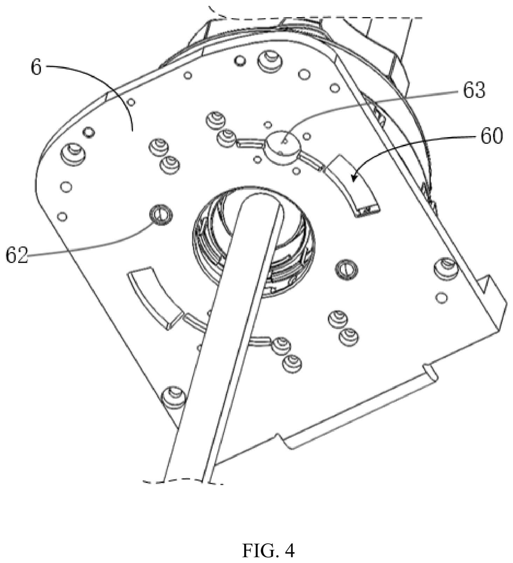

Referring to FIGS. 3 and 4, in some embodiments of the present application, the drive device further includes a base 6. The outer housing 1 is fixed to the base 6, and the sterile barrier penetrates through the base 6 and is clamped with the base 6.

For example, the outer housing 1 is fixed to a top wall of the base 6 in a manner such as clamping, welding, bonding, or bolt connection. The middle of the base 6 is provided with a through hole coaxial and communicating with the first mounting cavity, a partial structure of the sterile barrier penetrates through the through hole, is inserted into the first mounting cavity, and is fitted to another structure of the sterile barrier in an inserted manner to fix the sterile barrier in the inner housing 2, thereby isolating the laparoscope from the drive assembly 3 and ensuring that the laparoscope 7 is in the sterile environment.

In some embodiments of the present application, the drive device further includes a plunger 62 and a detector 63. The plunger 62 is disposed on the base 6 and inserted into the sterile barrier. The detector 63 is disposed on the base 6 and corresponds to the sterile barrier to detect a position of the sterile barrier relative to the base 6.

For example, the plunger 62 includes two parts, a cylinder and a spring, and is movably connected to a bottom wall of the base 6 through the spring, and the sterile barrier is provided with a groove for the plunger 62 to be inserted into. When the sterile barrier is mounted in place, the cylinder extends into the groove to fix the position of the sterile barrier. At this time, the sterile barrier remains clamped with the base 6 so that the sterile barrier is mounted and fixed.

The detector 63 may be a contact sensor such as a microswitch or a non-contact sensor such as an infrared sensor as long as it can detect the position of the sterile barrier relative to the base 6. To facilitate the mounting of the detector 63, the bottom wall of the base 6 is provided with a mounting hole for mounting the detector 63. An arc-shaped slot is opened through the bottom wall of the base 6 and along a mounting path of the sterile barrier, where the mounting hole is located in the middle of the arc-shaped slot. It is to be understood that one of the detector 63 or the plunger 62 may be provided or both the detector 63 and the plunger 62 may be provided. In this embodiment, two plungers 62 are provided opposite each other and two detectors 63 are provided opposite each other.

During mounting, the sterile barrier is pressed against the cylinder of the plunger 62 so that the spring is in an elastically deformed state. When the sterile barrier is clamped with the base 6, the plunger 62 is just aligned with the groove on the sterile barrier, and the plunger 62 is inserted into the groove to fix the position of the sterile barrier. At this time, the sterile barrier can correspond to the mounting hole to trigger the detector 63 so that the detector 63 can detect the position of the sterile barrier. Thus, whether the sterile barrier is mounted in place can be automatically detected, which not only helps to reduce a difficulty in using the whole drive device but also improves the automation of the whole drive device.

Referring to FIGS. 2 and 3, in some embodiments of the present application, the drive assembly 3 includes a hollow slim electric motor to reduce the length of an overlap between the mounted laparoscope 7 and the drive assembly 3 in the axial direction of the laparoscope 7. For example, an outer sidewall of the outer housing 1 may be provided with a conduction opening 10, making it convenient to conduct the drive assembly 3 with a power supply via a line. The drive assembly 3 includes a stator 30 and a rotor 31. The stator 30 is fixed to the inner wall of the outer housing 1. The rotor 31 is sleeved on the outer wall of the inner housing 2, coaxial with the stator 30, and axially aligned with the stator 30 in height to form the hollow slim electric motor. The stator 30 is located below the bearing 5 and fixed on the inner wall of the outer housing 1 through a corresponding fixing structure, the rotor 31 is annularly disposed and coaxial with the stator 30, the rotor 31 is fixed on the outer wall of the inner housing 2, and the height of the rotor 31 is lower than the height of the second mounting cavity, thereby reducing an occupied axial space in the second mounting cavity and correspondingly reducing the length of the overlap between the laparoscope 7 and the drive assembly 3 in the axial direction.

The stator 30 and the rotor 31 are directly disposed in the second mounting cavity to form the hollow slim electric motor so that the drive assembly 3 is relatively low in height and occupies a reduced axial space. Thus, a space inside the drive device can be more effectively and reasonably utilized so that the overall structure of the drive device can be more compact; moreover, the overall size of the drive device can be reduced to reduce a possibility of interference with the route of the laparoscope 7. Meanwhile, due to the hollow structure of the hollow slim electric motor, a snap-fit fixing structure between the sterile barrier and the inner housing 2 can be disposed at a position close to the base 6, which is conducive to reducing a loss of the axial length of the laparoscope 7 inserted into the drive device during mounting.

In some embodiments of the present application, the drive assembly 3 further includes an encoder magnetic ring 32 and an encoder read head 33. The encoder magnetic ring 32 is disposed on the inner housing 2. The encoder read head 33 is disposed on the outer housing 1 and corresponds to the encoder magnetic ring 32. For example, the encoder magnetic ring 32 is fixed to the bottom of the inner housing 2, and the encoder read head 33 is fixed at a corresponding position at the bottom of the outer housing 1. As the inner housing 2 rotates, the encoder magnetic ring 32 corresponds to the encoder read head 33 to detect a rotation angle of the inner housing 2 in real time. To avoid interference, a shield ring 34 is provided in the second mounting cavity and between the encoder magnetic ring 32 and the rotor 31 to prevent an electromagnetic field of the drive assembly 3 from interfering with the encoder read head 33.

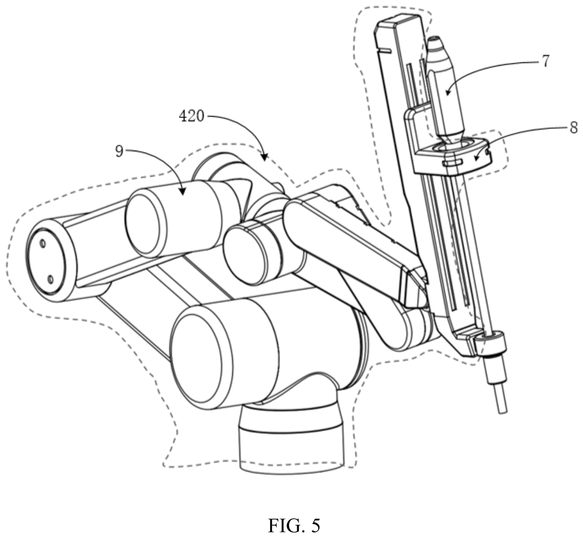

Referring to FIG. 5, in some embodiments of the present application, the drive device further includes a box 8. The box 8 is configured to be connected to a robotic arm 9, and the outer housing 1 is disposed in the box 8. For example, the box 8 may be L-shaped, a vertical arm is configured to be slidably connected to a lifting arm of the robotic arm 9, and the drive device is disposed in an inner wall of a horizontal arm.

Referring to FIGS. 1 to 5, the present application further provides a sterile barrier mounted in the drive device according to any preceding embodiment to fixedly connect the laparoscope 7 to the inner housing 2 and isolate the laparoscope 7 from the inner housing 2. The sterile barrier includes an isolation assembly 4 and a sterile drape 420, where the isolation assembly 4 penetrates through the first mounting cavity and is fixed relative to the inner housing 2. The sterile drape 420 is disposed on the isolation assembly 4.

For example, the isolation assembly 4 may be composed of an upper part and a lower part, the upper part and the lower part are inserted into the first mounting cavity from two sides of the drive device one to one, and the upper part and the lower part extend into the first mounting cavity to be fitted to each other in an inserted manner and clamped with the drive device, to enable the isolation assembly 4 to be quickly mounted to and detached from the drive device. A sterile channel is formed in the isolation assembly 4 for the laparoscope 7 to penetrate through to be mounted. A mounting ring-shaped plate 42 may be rotatably connected to the isolation assembly 4, the sterile drape 420 is fixed to the mounting ring-shaped plate 42, and the sterile drape 420 covers the whole box 8 and the whole robotic arm 9 to form the desired sterile environment. A material and a size of the sterile drape 420 may be designed according to the actual length of the robotic arm 9, which are not limited in the present application.

During use of the sterile barrier, the isolation assembly 4 is directly mounted in the first mounting cavity and connected to the inner housing 2 so that the laparoscope 7 is isolated from the drive assembly 3, and the requirement of the laparoscope 7 for the sterile environment is satisfied. The drive assembly 3 may drive the inner housing 2 and the isolation assembly 4 to rotate to directly drive the laparoscope 7 to rotate so that an adapter is no longer required for connecting the laparoscope 7 to the drive assembly 3, and a mounting structure of the laparoscope 7 is relatively simple, facilitating the quick mounting and detachment of the laparoscope 7.

In some embodiments of the present application, the isolation assembly 4 includes a first sterile shell 40 and a second sterile shell 41. The first sterile shell 40 is inserted into the first mounting cavity along a first side of the inner housing 2 and detachably connected to the inner housing 2. The second sterile shell 41 is inserted into the first mounting cavity along a second side of the inner housing 2 and inserted into and fitted to the first sterile shell 40 to fix the first sterile shell 40 to the inner housing 2. The laparoscope 7 penetrates through the first sterile shell 40 and the second sterile shell 41.

For example, the first sterile shell 40 is stepped, a large end face of the first sterile shell 40 abuts against a top wall of the mounting ring-shaped plate 42, and a small end face of the first sterile shell 40 penetrates through the mounting ring-shaped plate 42 and is inserted into the first mounting cavity. An end of the first sterile shell 40 inserted into the first mounting cavity is conical, and the end is clamped to a groove on an outer sidewall of the laparoscope 7 so that the first sterile shell 40 is fixed to the laparoscope 7 through clamping. Block-like clamping structures may be further provided on an outer sidewall of the first sterile shell 40, and the inner housing 2 may be provided with slots clamped with the clamping structures so that the first sterile shell 40 can be clamped with the inner housing 2 when the first sterile shell 40 is inserted into the first mounting cavity. It is to be understood that the inner housing 2 may be provided with clamping structures, and slots are correspondingly defined on the outer sidewall of the first sterile shell 40.

The second sterile shell 41 is inserted into the first mounting cavity from the lower end of the outer housing 1, and the second sterile shell 41 is inserted into and fitted to the first sterile shell 40 so that the first sterile shell 40 can abut against the inner housing 2, ensuring connection strength between the first sterile shell 40 and the inner housing 2, ensuring that the first sterile shell 40 is not detached axially, and enabling the inner housing 2 to smoothly drive the first sterile shell 40 to rotate.

During mounting of the laparoscope 7, the lower end of the first sterile shell 40 is inserted into the first mounting cavity and the second sterile shell 41 is correspondingly inserted to fix the first sterile shell 40 to the inner housing 2, then the laparoscope 7 is inserted into the first sterile shell 40, and the first sterile shell 40 is clamped to the groove on the laparoscope 7 so that the laparoscope 7 is fixed in the first sterile shell 40 and can be quickly mounted. In the above mounting process, the mounting of the adapter does not need to be considered, the mounting process is simpler, and the isolation assembly 4 is directly disposed in the inner housing 2 and the drive assembly 3 is disposed between the outer housing 1 and the inner housing 2 so that the layout is more reasonable and compact, the overall volume and size of the drive device including the sterile barrier are reduced, and the possibility of interference with the route of the laparoscope 7 is effectively reduced. Meanwhile, the first sterile shell 40 is detachably connected to the inner housing 2. When different types of laparoscopes 7 are replaced, only the first sterile shell 40 that can be clamped to the laparoscope 7 needs to be replaced so that different types of laparoscopes 7 can be fixed to the inner housing 2, and the drive device can be compatible with different types of laparoscopes 7.

Referring to FIGS. 2 and 3, in some embodiments of the present application, the first sterile shell 40 includes a first sleeve 400 and clamping blocks 401. The first sleeve 400 is inserted into the first mounting cavity. The clamping blocks 401 are disposed on an outer sidewall of the first sleeve 400, where the inner housing 2 is provided with clamping slots clamped with the clamping blocks 401. The clamping blocks 401 include a first clamping block and a second clamping block having a greater cross-sectional area than the first clamping block, and the clamping slots include a first clamping slot 20 and a second clamping slot 21, where the first clamping slot 20 has a smaller cross-sectional area than the second clamping slot 21, the first clamping block is inserted into and fitted to the first clamping slot 20, and the second clamping block is inserted into and fitted to the second clamping slot 21.

For example, the first clamping slot 20 and the second clamping slot 21 are both defined on the upper end face of the inner housing 2, the clamping blocks 401 are fixed on the outer sidewall of the first sleeve 400, and each clamping slot corresponds to one clamping block 401, where a size of the clamping block 401 is adapted to a size of the corresponding clamping slot, that is, the clamping block 401 inserted into the first clamping slot 20 has the smaller cross-sectional area. It is to be understood that the number of clamping blocks 401 and the number of clamping slots may be 2, 3, 4, or greater, which are designed according to the sizes of the first sleeve 400 and the inner housing 2. In this embodiment, one first clamping slot 20 and one second clamping slot 21 are provided, and the two clamping slots are distributed on the inner housing 2 and spaced by 180° to effectively implement a foolproof function.

The clamping slots and the clamping blocks 401 are designed so that when the first sleeve 400 is inserted into the first mounting cavity, the inner housing 2 is clamped with and fitted to the first sterile shell 40, and the inner housing 2 can conveniently drive the first sterile shell 40 to rotate during rotation. The first clamping slot 20 and the second clamping slot 21 having different cross-sectional areas make it convenient to specify a mounting direction of the first sterile shell 40 when the first sterile shell 40 is inserted, ensuring that the first sterile shell 40 is mounted in a correct direction.

Referring to FIGS. 1 and 2, in some embodiments of the present application, the first sterile shell 40 further includes a snap-fit 402. The snap-fit 402 is disposed on a side of the first sleeve 400 inserted into the first mounting cavity, where the inner housing 2 is provided with an interface 22 clamped with the snap-fit 402 to prevent the first sleeve 400 from moving along an axial direction of the first mounting cavity.

For example, since the first sterile shell 40 is stepped, the snap-fit 402 is disposed on an end face facing the second sterile shell 41, and the snap-fit 402 includes an arc-shaped plate and a clasp, where the arc-shaped plate extends along an axial direction of the first sterile shell 40, and the clasp is formed at an end facing the second sterile shell 41. One snap-fit 402 may be provided, or multiple snap-fits 402 may be distributed at intervals along a circumferential direction of the first sterile shell 40. In this embodiment, four snap-fits 402 are provided.

The snap-fit 402 is disposed so that after the first sterile shell 40 is inserted into the first mounting cavity, the snap-fit 402 is clamped with the interface 22, and the first sterile shell 40 is clamped to the inner housing 2 to prevent the first sterile shell 40 from moving in an axial direction of the inner housing 2. Meanwhile, the interface 22 is disposed close to the lower end of the inner housing 2 so that the mating of the snap-fit 402 with the interface 22 is close to the lower side of the drive device, the laparoscope 7 is mounted deeper in the drive device, and the loss of the length of the laparoscope 7 can be better reduced, which is conducive to ensuring that the laparoscope 7 maintains a relatively large movement route.

In some embodiments of the present application, a deformation gap is formed between the snap-fit 402 and the first sleeve 400, and the second sterile shell 41 is partially inserted into the deformation gap.

For example, the snap-fit 402 may be made of an elastic material or another material with a deformation capability, such as plastic, so that the snap-fit 402 has a certain deformation capability. A certain spacing is maintained between an inner sidewall of the snap-fit 402 and the outer sidewall of the first sleeve 400 to form the deformation gap, and the second sterile shell 41 can be partially inserted into the deformation gap to prevent the deformation of the snap-fit 402.

When the first sterile shell 40 is inserted into the first mounting cavity, the snap-fit 402 is pressed to deform towards the deformation gap. After the snap-fit 402 is clamped with the interface 22, the deformation of the snap-fit 402 disappears. As the second sterile shell 41 is inserted and fills the deformation gap, the deformation tendency of the snap-fit 402 is stopped, and the snap-fit 402 without deformation can be firmly kept clamped with the interface 22 so that the first sterile shell 40 can be smoothly fixed in the first mounting cavity. Can the first sterile shell 40 be detached only after the second sterile shell 41 is removed, to ensure the overall mounting stability of the drive device.

Referring to FIGS. 1 and 2, in some embodiments of the present application, the second sterile shell 41 includes a sterile plate 410, a second sleeve 411, and a top ring 412. The second sleeve 411 and the top ring 412 are disposed on the sterile plate 410, the second sleeve 411 is partially inserted into the first sleeve 400, the top ring 412 is annularly disposed outside the second sleeve 411 and inserted into the deformation gap, and an outer sidewall of the top ring 412 abuts against the inner sidewall of the snap-fit 402.

For example, the sterile plate 410, the second sleeve 411, and the top ring 412 may be integrally formed, the sterile plate 410 may be a circular plate, the second sleeve 411 and the top ring 412 are both coaxial with the sterile plate 410, and the outer diameter of the second sleeve 411 is smaller than the inner diameter of the first sleeve 400. An inner sidewall of the top ring 412 is fitted to the outer sidewall of the first sleeve 400, and the outer sidewall of the top ring 412 abuts against the inner sidewall of the snap-fit 402 so that as the second sleeve 411 is inserted into the first sleeve 400, the top ring 412 is inserted into the deformation gap and presses the snap-fit 402, and the snap-fit 402 remains clamped with the corresponding interface 22.

When the second sterile shell 41 is mounted, the second sleeve 411 is correspondingly inserted into the first sleeve 400. When the top ring 412 is inserted into the deformation gap, the second sterile shell 41 is inserted into and fitted to the first sterile shell 40, and the deformation of the snap-fit 402 is prevented, ensuring that the first sterile shell 40 is fixed to the inner housing 2 through clamping.

In some embodiments of the present application, the second sterile shell 41 further includes insertion blocks 413. The insertion blocks 413 are disposed on the sterile plate 410, clamping cavities are formed inside the base 6, clamping gaps 60 communicating with the clamping cavities are defined on a surface of the base 6, the insertion blocks 413 are clamped with the clamping cavities through the clamping gaps 60, and the insertion blocks 413 slide into clamping cavities.

The second sleeve 411 penetrates through the through hole of the base 6 and is inserted into the first mounting cavity. The insertion blocks 413 are disposed on an edge of the sterile plate 410 and may be integrally formed with the sterile plate 410. A clamping gap 60 is defined according to a shape of an insertion block 413. In this embodiment, the insertion block 413 is ┌-shaped, a vertical arm of the insertion block 413 is connected to the sterile plate 410, and a horizontal arm of the insertion block 413 can be correspondingly inserted into the clamping gap 60, and the sterile plate 410 is rotated such that the bottom wall of the base 6 is inserted between the horizontal arm of the insertion block 413 and the sterile plate 410 so that the sterile plate 410 can be clamped with the base 6.

In some embodiments of the present application, two insertion blocks 413 are provided and located on two sides of the second sleeve 411 one to one, and two corresponding clamping gaps 60 are provided. It is to be understood that in other embodiments, the number of insertion blocks 413 may be 3 or greater and may be designed, for example, according to the sizes of the base 6 and the sterile plate 410.

When the second sterile shell 41 is mounted, both the second sleeve 411 and the top ring 412 penetrate through the through hole of the base 6 and are inserted into the first mounting cavity, the insertion blocks 413 are inserted into the clamping gaps 60, and the second sleeve 411 is driven to spin so that the insertion blocks 413 are inserted into the clamping cavities of the base 6, and the sterile plate 410 can be fixed to the base 6. The sterile plate 410 is provided with a groove for the plunger 62 to be inserted into. After the second sterile shell 41 is mounted in place, the cylinder of the plunger 62 extends into the groove, and the insertion block 413 simultaneously triggers the detector 63. After the detector 63 detects that the second sterile shell 41 is mounted in place, it can be determined that the mounting of the isolation assembly 4 is completed. When the isolation assembly 4 needs to be detached, pressure is applied to two handles disposed on a bottom surface of the sterile plate 410, and the sterile plate 410 is rotated reversely so that the plunger 62 can be detached from the groove on the sterile plate 410, and the second sterile shell 41 can be quickly detached. After the second sterile shell 41 is removed, the first sterile shell 40 can be taken out.

A surgical robot according to an embodiment of the present application includes a robotic arm 9 and the drive device and the sterile barrier according to any preceding embodiment. The drive device is disposed on the robotic arm 9, and the sterile barrier is disposed in the drive device to enable the laparoscope 7 to be detachably connected to and remain isolated from the drive device.

During use of the surgical robot, the sterile barrier and the drive device are disposed so that the laparoscope 7 can not only maintain a relatively large route but also rotate by an angle greater than 360°, ensuring that the surgical robot can be normally used, reducing a possibility of an effect on surgery, and improving the success rate of the surgery.

In the first aspect, the second mounting cavity is formed between the inner housing and the outer housing to provide a mounting space for the drive assembly so that the drive assembly can be mounted inside the drive device. After the laparoscope is directly mounted into the first mounting cavity through the sterile barrier, the drive assembly may drive the inner housing to rotate to directly drive the laparoscope to rotate so that the adapter is no longer required for mounting the laparoscope. Therefore, during use of the drive device, the overall volume and size of the drive device are effectively reduced, and the drive device is prevented from interfering with the movement route of the laparoscope, thereby satisfying that the laparoscope has a relatively large movement route.

In the second aspect, during use of the sterile barrier, the isolation assembly is directly mounted in the first mounting cavity and connected to the inner housing so that the laparoscope is isolated from the drive assembly, and the requirement of the laparoscope for the sterile environment is satisfied. The drive assembly may drive the inner housing and the isolation assembly to rotate to directly drive the laparoscope to rotate so that the adapter is no longer required for connecting the laparoscope to the drive assembly, and the mounting structure of the laparoscope is relatively simple, facilitating the quick mounting and detachment of the laparoscope.

In the third aspect, during use of the surgical robot, the sterile barrier and the drive device are disposed so that the laparoscope can not only maintain a relatively large route but also rotate by an angle greater than 360°, ensuring that the surgical robot can be normally used, reducing the possibility of an effect on the surgery, and improving the success rate of the surgery.

## Claims

1. A drive device configured to drive a laparoscope (7) to rotate and comprising:
an outer housing (1);
an inner housing (2) disposed in the outer housing (1) and rotatably connected to the outer housing (1) via a bearing (5), wherein the inner housing (2) forms a first mounting cavity configured for mounting a sterile barrier, the laparoscope (7) penetrates through the sterile barrier, and a second mounting cavity is formed between an outer wall of the inner housing (2) and an inner wall of the outer housing (1); and
a drive assembly (3) disposed in the second mounting cavity and connected to the inner housing (2) to drive the inner housing (2) to rotate.

2. The drive device according to claim 1, wherein the outer housing (1), the inner housing (2), and the drive assembly (3) are all annular to enable the drive assembly (3) to drive the inner housing (2) to rotate by an angle greater than 360°.

3. The drive device according to claim 1, further comprising:
a base (6), wherein the outer housing (1) is fixed to the base (6), and the sterile barrier penetrates through the base and is clamped with the base.

4. The drive device according to claim 3, further comprising at least one of:
a plunger (62) disposed on the base (6) and configured to be inserted into the sterile barrier; or
a detector (63) disposed on the base (6) and corresponding to the sterile barrier to detect a position of the sterile barrier relative to the base (6).

5. The drive device according to claim 1, wherein the drive assembly (3) comprises a hollow electric motor to reduce a length of an overlap between the mounted laparoscope (7) and the drive assembly (3) in an axial direction of the laparoscope (7).

6. The drive device according to claim 5, wherein the drive assembly (3) further comprises:
an encoder magnetic ring (32) disposed on the inner housing (2); and
an encoder read head (33) disposed on the outer housing (1) and corresponding to the encoder magnetic ring (32).

7. The drive device according to claim 1, further comprising:
an inner pressure plate (51) fixedly connected to an inner ring of the bearing (5) and the inner housing (2) separately; and
an outer pressure plate (50) fixedly connected to an outer ring of the bearing (5) and the outer housing (1) separately.

8. The drive device according to any one of claims 1 to 7, further comprising:
a box (8) configured to be connected to a robotic arm (9), wherein the outer housing (1) is disposed in the box (8).

9. A sterile barrier mounted in the drive device according to any one of claims 1 to 8 to fixedly connect the laparoscope (7) to the inner housing (2) and isolate the laparoscope (7) from the inner housing (2), and the sterile barrier comprising:
an isolation assembly (4) penetrating through the first mounting cavity and fixed relative to the inner housing (2); and
a sterile drape (420) disposed on the isolation assembly (4).

10. The sterile barrier according to claim 9, wherein the isolation assembly (4) comprises:
a first sterile shell (40) inserted into the first mounting cavity along a first side of the inner housing (2) and detachably connected to the inner housing (2); and
a second sterile shell (41) inserted into the first mounting cavity along a second side of the inner housing (2) and inserted into and fitted to the first sterile shell (40) to fix the first sterile shell (40) to the inner housing (2);
wherein the laparoscope (7) penetrates through the first sterile shell (40) and the second sterile shell (41).

11. The sterile barrier according to claim 10, wherein the first sterile shell (40) comprises:
a first sleeve (400) inserted into the first mounting cavity; and
clamping blocks (401) disposed on an outer sidewall of the first sleeve (400);
wherein the inner housing (2) is provided with clamping slots clamped with the clamping blocks (401).

12. The sterile barrier according to claim 11, wherein the clamping blocks (401) comprise a first clamping block and a second clamping block having a greater cross-sectional area than the first clamping block, and the clamping slots comprise a first clamping slot (20) and a second clamping slot (21), wherein the first clamping slot (20) has a smaller cross-sectional area than the second clamping slot (21), the first clamping block is inserted into and fitted to the first clamping slot (20), and the second clamping block is inserted into and fitted to the second clamping slot (21).

13. The sterile barrier according to claim 11, wherein the first sterile shell (40) further comprises:
a snap-fit (402) disposed on a side of the first sleeve (400) inserted into the first mounting cavity;
wherein the inner housing (2) is provided with an interface (22) clamped with the snap-fit (402) to prevent the first sleeve (400) from moving along an axial direction of the first mounting cavity.

14. The sterile barrier according to claim 13, wherein a deformation gap is formed between the snap-fit (402) and the first sleeve (400), and the second sterile shell (41) is partially inserted into the deformation gap; and
the second sterile shell (41) comprises:
a sterile plate (410), wherein a second sleeve (411) and a top ring (412) are provided on the sterile plate (410), the second sleeve (411) is partially inserted into the first sleeve (400), the top ring (412) is annularly disposed outside the second sleeve (411) and inserted into the deformation gap, and an outer sidewall of the top ring (412) abuts against an inner sidewall of the snap-fit (402).

15. A surgical robot, comprising:
a robotic arm (9);
the drive device according to any one of claims 1 to 8, which is disposed on the robotic arm (9); and
the sterile barrier according to any one of claims 9 to 14, which is disposed in the drive device to enable the laparoscope (7) to be detachably connected to and remain isolated from the drive device.
